# EUROPEAN PATENT APPLICATION

(11) **EP 1 777 635 A1**
(43) Date of publication of application: **25.04.2007**
(21) Application number: 05109665.9
(22) Date of filing: 18.10.2005
(51) Int. Cl.: G06F 19/00

(54) **Method for generating an electronic patient record**

(71) Applicant: Quadrat, 2640 Mortsel (BE)
(72) Inventor: Bonte, Pierre, 2640, Mortsel (BE)
(74) Representative: Verbrugghe, Anne Marie L.

(57) **Abstract**

Method for generating an electronic patient record comprising objects that may be linked and for which patient-related data can be entered in a data base. A time stamp is added to the patient-related data and/or to a link at the time of entry or revision of the patient-related data or of the link.

## Description

### FIELD OF THE INVENTION

The present invention relates to a method for generating an electronic patient record.

### BACKGROUND OF THE INVENTION

An electronic patient record of a patient comprises a number of objects such as contact (i.e. patient visiting physician), examination, diagnosis, medication, reports etc. that are associated with patient identification data. These objects can be linked, i.e. a semantic meaning is adhered to the objects. For example an examination can be linked or reverse-linked to a diagnosis to express that an examination is an indication for a diagnosis (linked objects) or a diagnosis is made as a result of an examination (reverse-linked objects).

Different objects regarding one patient may be added to a patient's electronic record on different moments in time. For example as a result of a first visit of a patient to a physician (in electronic patient record referred to as 'a contact') a first examination is performed and the result of this examination leads to a first examination report which is entered in the patient's record.
This report can help the physician to come to a conclusion that he will validate there after.
A second visit of the patient may result in a review of the examination report which was entered to the electronic patient record. This review can result in another conclusion by the referring physician.
In the existing software packages for the generation of electronic patient records it is possible to enter different versions of a report or an object. It is however not possible to review the history of the decision making process. In this process not only the objects but also the links between objects play an important role.

It is therefor an object of the present invention to provide a method of generating an electronic patient record in which the above-mentioned shortcomings of the prior art are overcome.

### SUMMARY OF THE INVENTION

The above-mentioned objects are realized by a method having the specific features set out in claim 1.

Specific features for preferred embodiments of the invention are set out in the dependent claims.

Further advantages and embodiments of the present invention will become apparent from the following description and drawings.

### BRIEF DESCRIPTION OF THE DRAWINGS

Fig. 1 to fig. 7 are screen prints illustrating an electronic patient record according to an embodiment of the present invention.
Fig. 8 to fig. 11 are tables illustrating an embodiment of the technical implementation of the present invention,

### DETAILED DESCRIPTION OF THE INVENTION

The present invention is based on object linking and versioning of the data present in an EPR.

Each time medical objects in an EPR are linked a time stamp is provided. The linking of the objects is recorded in an object link table and the content of the objects is recorded in object content tables.

By providing a time stamp each time a link between objects in a medical record is established and by recording the content of the objects at that time in a table it is possible to recreate the history of the changes in an EPR.

Due to this combination of object linking and time stamping (versioning) all the data (historical and actual) are available at any point in time.

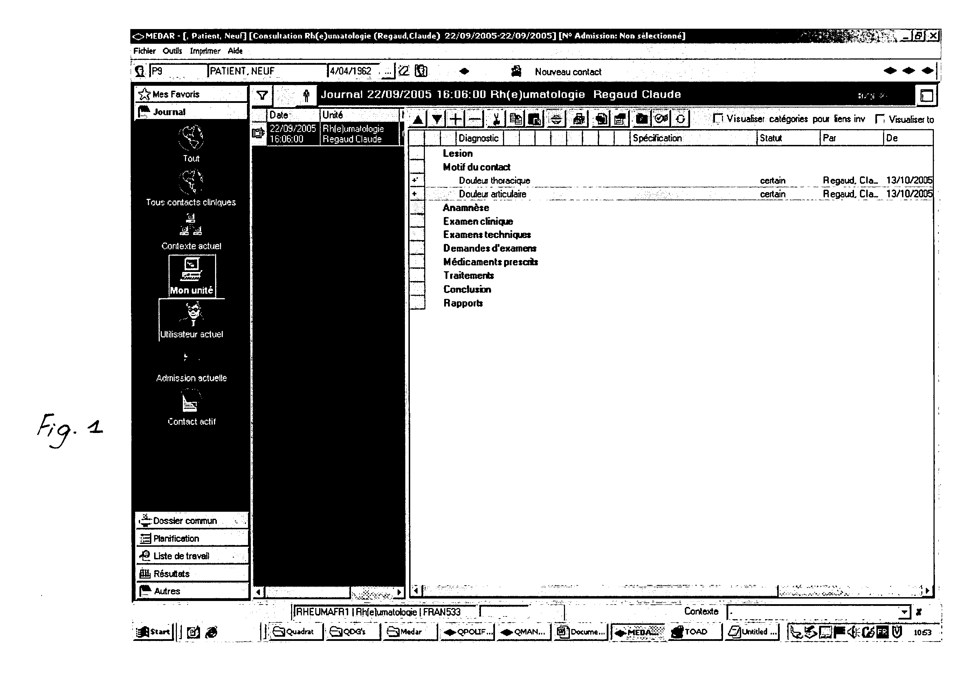

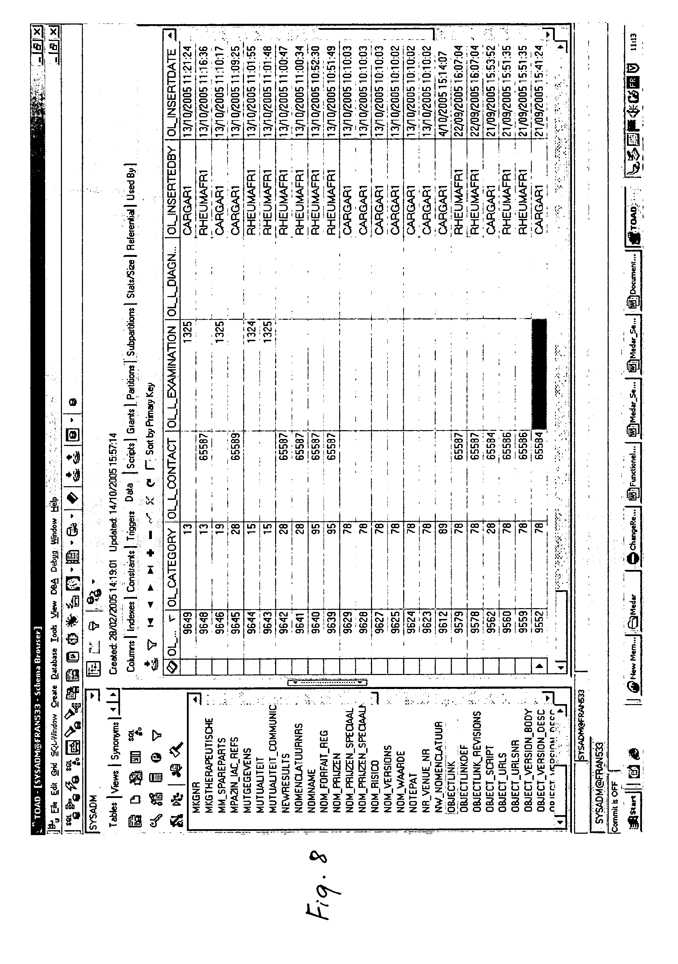

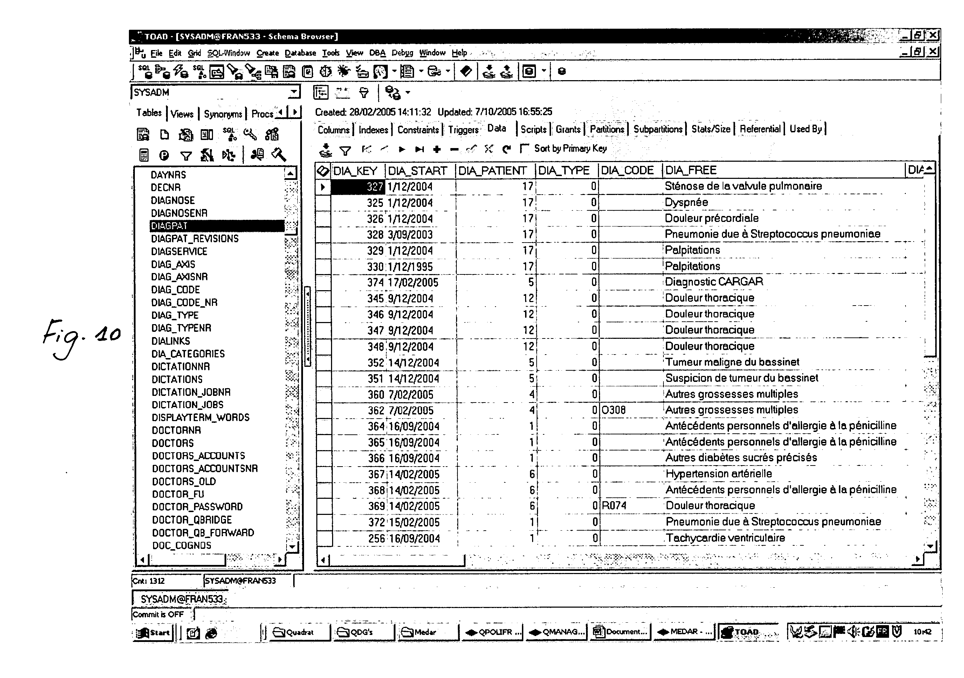

As shown in fig 1. in a first contact Patient N with multiple complaints (chest pain and arthritis) visits doctor Z (rheumatologist). During this contact doctor Z opens the EPR of patient N and enters the 'reason of contact' e.g. chest pain and arthritis. According to the method of the present invention the object 'contact' is linked to the object 'diagnosis'. The link is recorded in the 'object link table' (fig. 8) and the diagnosis is recorded in the 'object content table' e.g. 'diagpat table' (fig. 10), both receiving a time stamp. In a following step doctor Z orders two exams (ECG and RXThorax) for patient N.

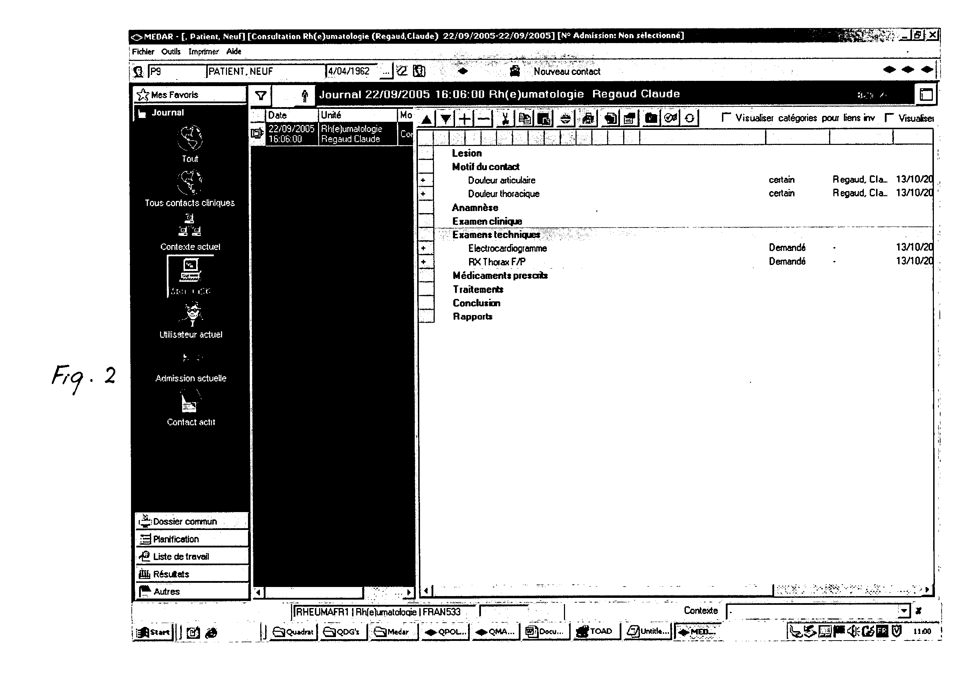

As shown in fig. 2 doctor Z enters the exam orders ECG and RXthorax for the diagnosis 'chest pain' directly in the EPR. In this step the object ' exam' is linked respectively to the object ' ECG' and to the object 'RXthorax'. The links and content are recorded in the respective tables and time stamps are provided. The patient is referred to doctor Y (cardiologist) for an ECG.

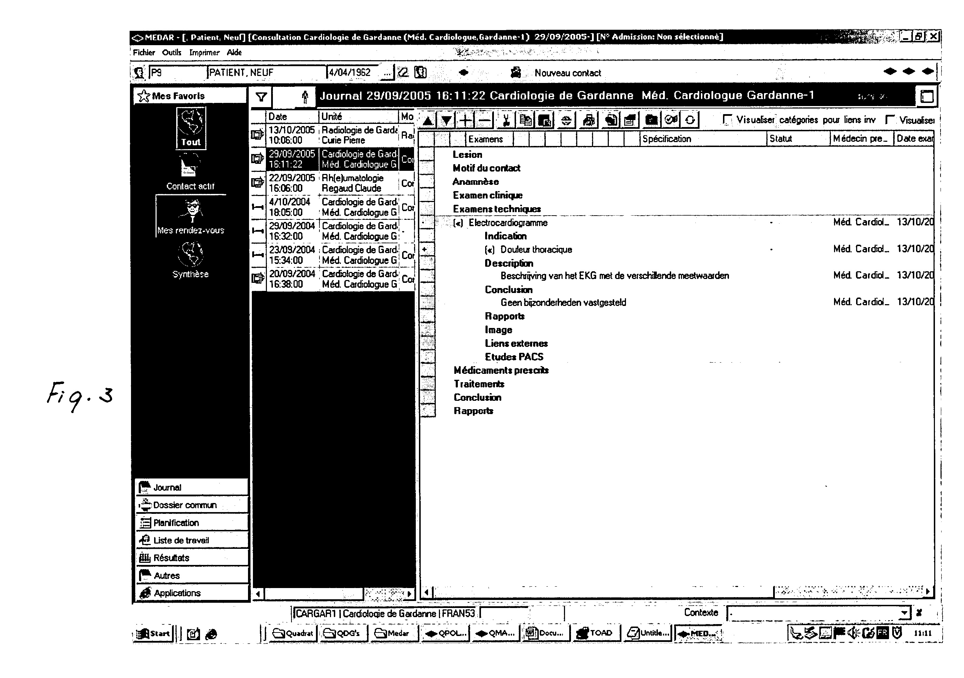

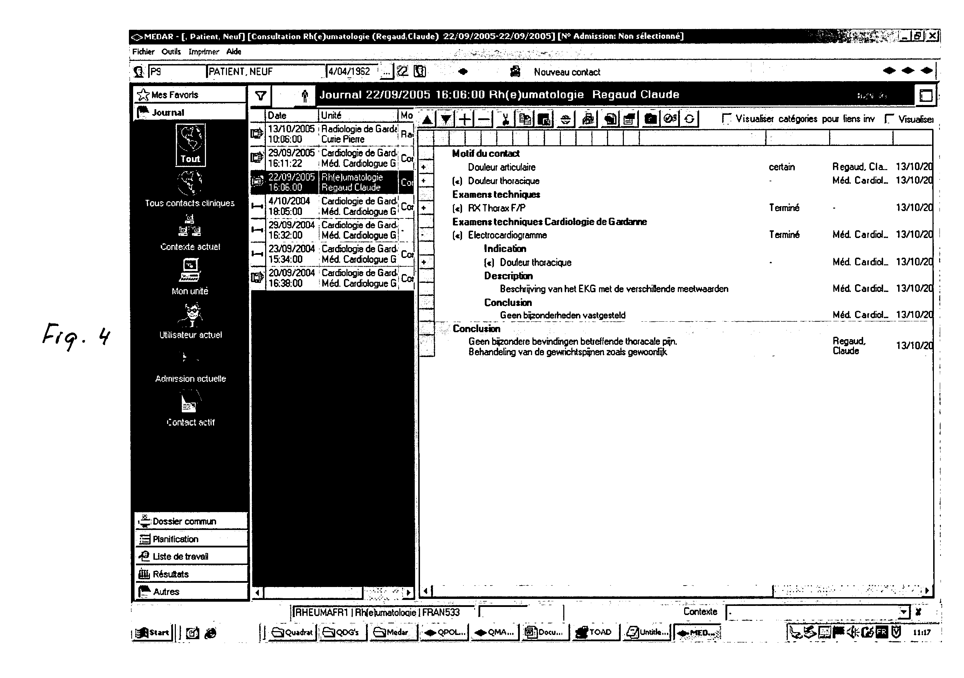

During the contact following the ECG exam as shown in fig. 3 doctor Y completes the EPR of patient N e.g. enters the indication for the exam (chest pain), a description (observations) and a conclusion (result of exam). Again according to the present invention the object 'exam' is linked to the objects 'indication', 'description' and 'conclusion'. The links and the content are recorded and time stamped. In the next step patient N returns to doctor Z for an evaluation of the exam results. During this contact as shown in fig. 4 doctor Z enters his conclusion, validates the entered data and prepares a report for the General Practitioner of patient N. In this step the object 'conclusion' is linked to the object 'contact' and the content and links are recorded. A time stamp is provided for the link and the content as well as for the validation act and report preparation.

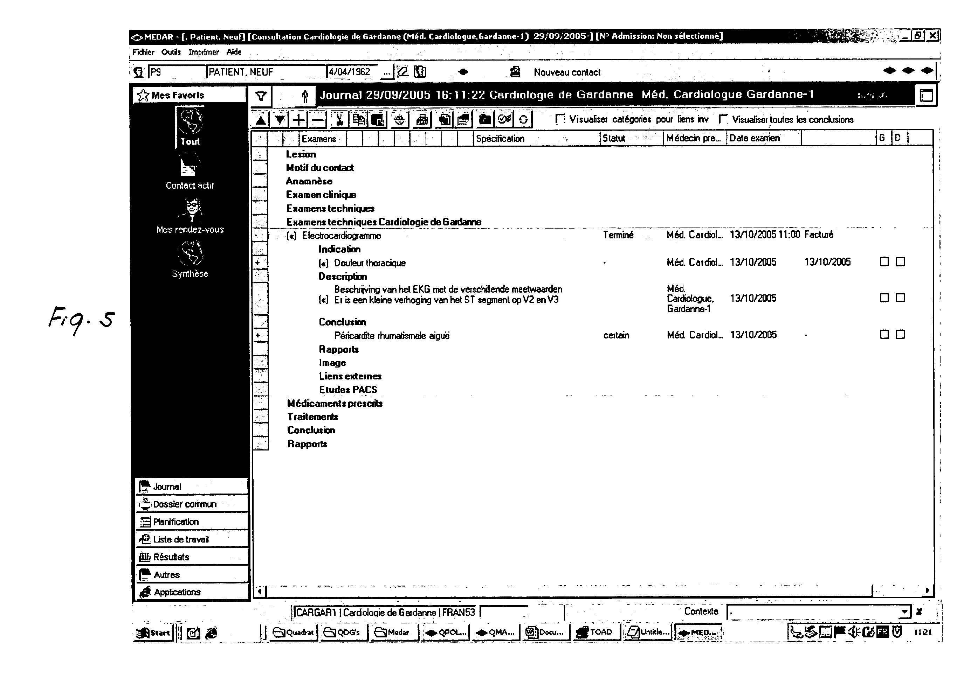

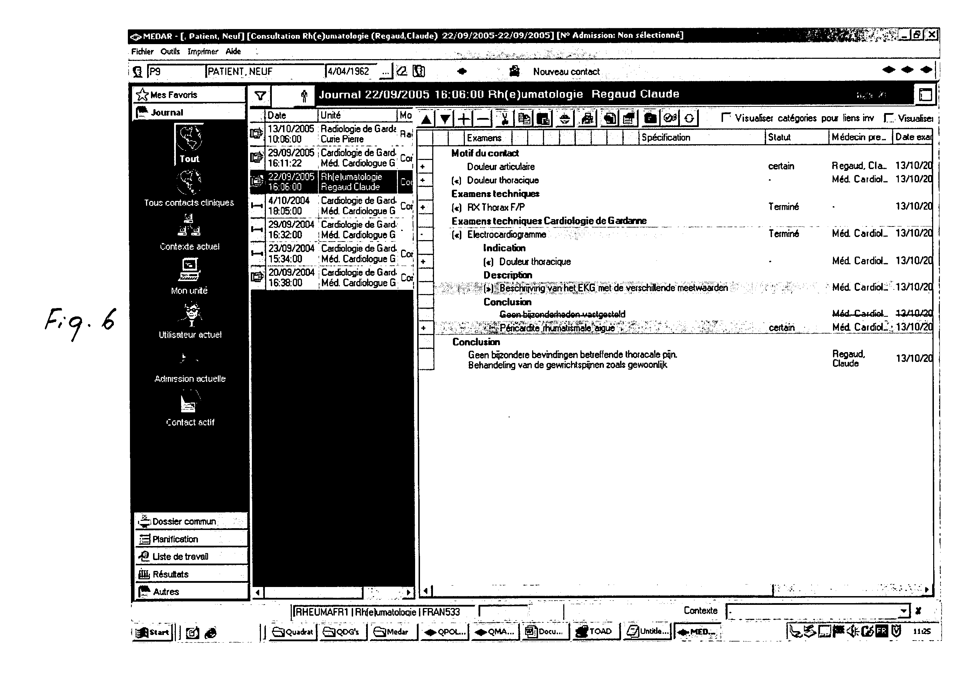

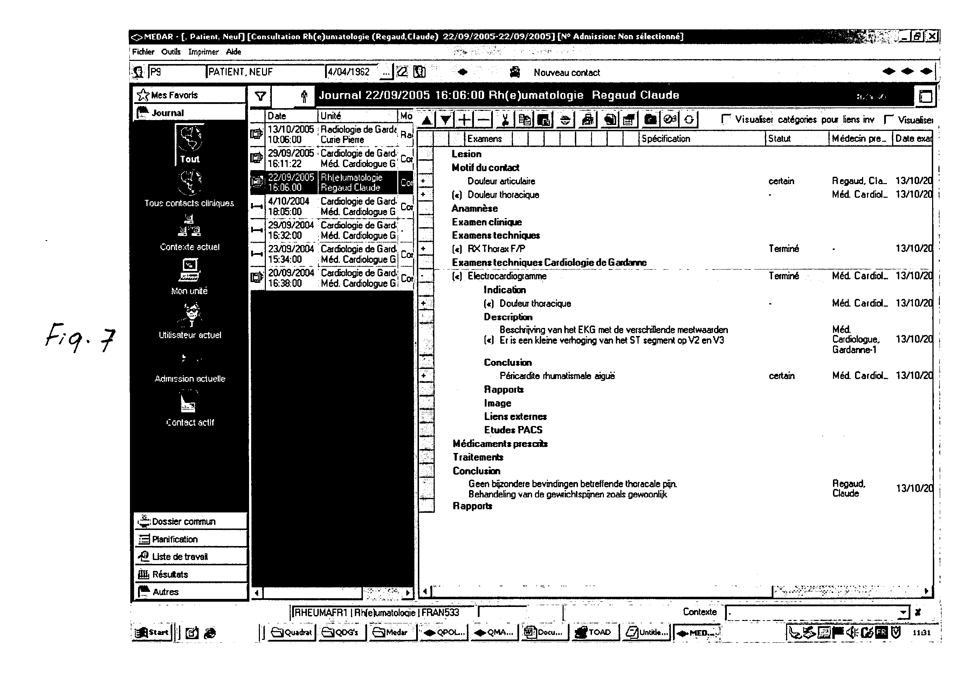

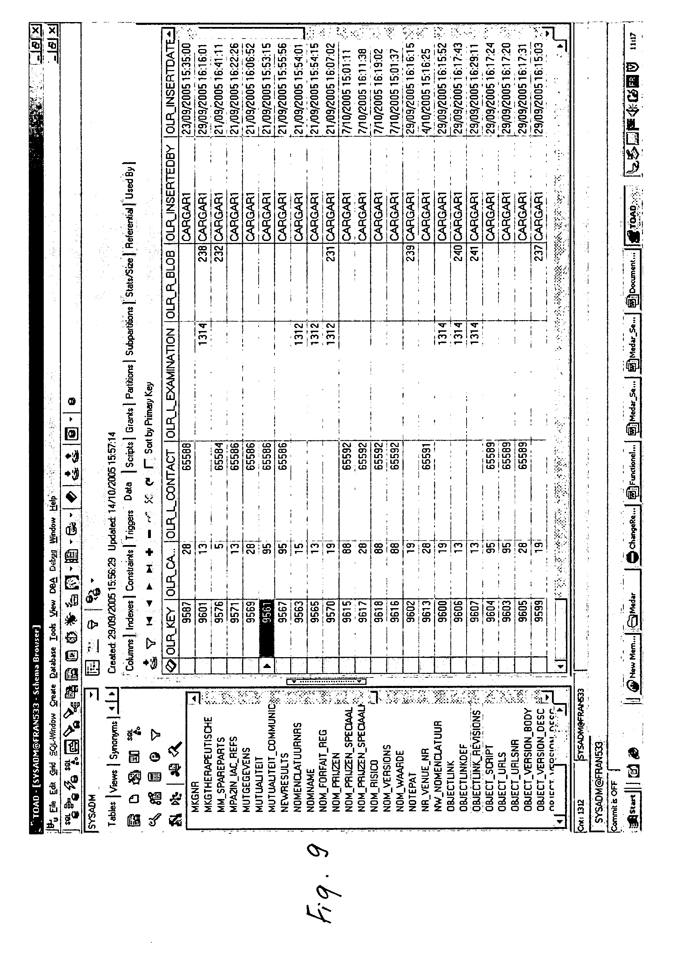

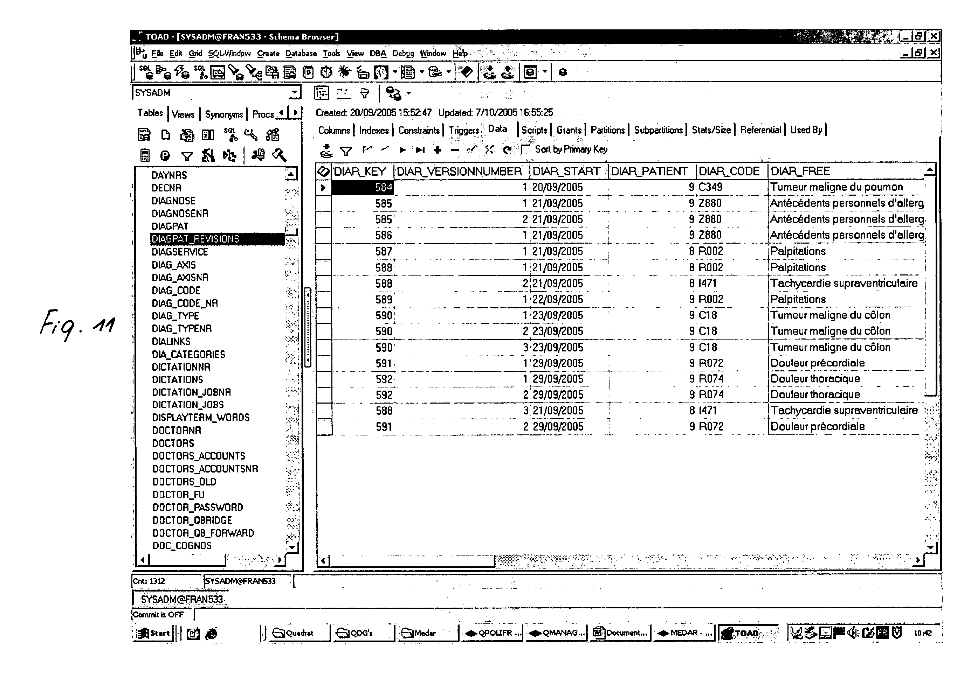

A few weeks later patient N returns with the same, but more severe complaints. The ECG results are re-evaluated by doctor Y and he corrects the description and conclusion in the exam part of the EPR of patient N as shown in fig. 5.
For these new entries, new links are established and time stamps are provided as described before. However, these links and object contents are stored in respectively the 'objectlink_revision table' and the 'object content_revision table' e.g. 'diagpat_revisions table' as shown in fig. 9 and fig. 11.
The new entries are shown in another colour then the previous entries (green). After the correction of doctor y, the new data can be evaluated by doctor Z (fig.6). Doctor Z can update the screen by selecting 'show at present time' in a drop-down menu. As shown in fig.7, this screen shows the actual status of the EPR and indicates the changes since the previous validation by >> or << (<< for earlier versions and >> for later versions). According to the method of the present invention changes in the content of the objects are stored and time stamped as well as changes in links. By storing these changes and time stamps the history of the changes in the content of the EPR of a patient can be reconstructed as shown in fig. 12. A time line and optional pointer can be constructed showing the changes in the content of an EPR. At each point in time the 'actual' status at that time can be reconstructed and compared with the present status. This gives the physician the opportunity to reconstruct the steps/ decisions that were taking in the process to come to the present conclusion. This time line and optional pointer can be implemented in the form of a scroll bar.

Having described in detail preferred embodiments of the current invention, it will now be apparent to those skilled in the art that numerous modifications can be made therein without departing from the scope of the invention as defined in the appending claims.■

## Claims

1. A method of generating an electronic patient record (EPR) comprising objects between which links may be established and for which patient-related data can be entered in a data base, **characterised in that** a time stamp is added to said patient-related data and/or to a link at the time of entry or revision of said patient-related data or of said link.

2. A method according to claim 1 wherein at least one table is created comprising
(1) object-content data regarding objects and time stamps expressing the time of entry of said object-content data in said table,
(2) object-content revision data regarding said objects and time stamps expressing the time of entry of revised object-content data in said table,
(3) object-link data regarding links established between objects and time stamps expressing the time of creation of said object-link data in said table,
(4) an object-link revision data regarding revisions of links between objects and time stamps expressing the time of entry of said revisions in said table.

3. A method of displaying the history of an electronic patient record (EPR) comprising the steps of
(1) displaying a time line,
(2) moving a pointer along the displayed time line,
(3) at each position of said pointer retrieving from at least one table comprising object-content data, object-content revision data, links between objects and revisions of links between objects, each with respective time stamp of data entry in said table, the data pertaining to the point of time indicated by the pointer on the time line,
(4) displaying the retrieved data.

4. A method according to claim 3 wherein data of said electronic patient record are retrieved up to and including the data pertaining to the point of time indicated by said pointer on said time line.

5. A method according to claim 3 wherein said time line and/or said pointer is implemented in the form of a scroll bar.
